# EUROPÄISCHE PATENTANMELDUNG

(11) **EP 3 669 926 A1**
(43) Veröffentlichungstag der Anmeldung: **24.06.2020**
(21) Anmeldenummer: 18213575.6
(22) Anmeldetag: 18.12.2018
(51) Int. Cl.: A61M 25/10, A61M 25/00, A61F 2/958, B23K 26/36

(54) **BALLONKATHETER SOWIE VERFAHREN UND VORRICHTUNG ZUR HERSTELLUNG DES BALLONKATHETERS**

(71) Anmelder: BIOTRONIK AG, 8180 Bülach (CH)
(72) Erfinder: Fargahi, Amir, 8180 Bülach (CH); Pantic, Dragica, 8051 Zürich (CH)
(74) Vertreter: Randoll, Sören

(57) **Zusammenfassung**

Die Erfindung betrifft einen Ballonkatheter (1), mit: einem Ballon (2), der einen Balloninnenraum (20) umgibt und einen distalen Endabschnitt (21) aufweist, und einem sich durch den Balloninnenraum (20) erstreckenden Innenschaft (3), der einen distalen Endabschnitt (31) aufweist, der sich an einen im Balloninnenraum (20) angeordneten (32) Abschnitt des Innenschafts (3) anschließt. Der im Balloninnenraum (20) angeordnete Abschnitt (32) des Innenschafts (3) weist mehrere übereinander liegende und miteinander verbundene Materialschichten auf, die ein Lumen des Innenschafts (3) umgeben, wohingegen der distale Endabschnitt (31) des Innenschafts (3) einschichtig ausgebildet ist. Weiterhin betrifft die Erfindung ein Verfahren und eine Vorrichtung (100) zur Herstellung des Ballonkatheters (1).

## Beschreibung

Die Erfindung betrifft einen Ballonkatheter sowie ein Verfahren und eine Vorrichtung zur Herstellung eines solchen Ballonkatheters.

Bei der Herstellung von Ballonkathetern mit einem mehrschichtigen Innenschaft hat sich das Verschweißen des Ballons mit dem distalen Endabschnitt als besonders herausfordernd erwiesen, da es hierbei aufgrund von thermomechanischen Belastungen zur Delamination von innenliegenden Materialschichten im Bereich der Schweißverbindung kommen kann. Dies ist insofern problematisch, da eine solche Delamination zur Blockade des Führungsdrahtlumens des Innenschafts führen kann.

Hiervon ausgehend liegt der vorliegenden Erfindung die Aufgabe zugrunde, einen Ballonkatheter bzw. ein Verfahren zur Herstellung des Ballonkatheters bereitzustellen, der keine Delamination eines mehrschichtigen Innenschaftes im Schweißbereich des Ballons aufweist.

Dieses Problem wird durch einen Ballonkatheter gelöst, dessen Aufbau so vorgeschlagen wird, dass dieser folgende Bauteile umfasst:
- einem Ballon, der einen Balloninnenraum umgibt und einen distalen Endabschnitt aufweist, und
- einem sich durch den Balloninnenraum erstreckenden Innenschaft, der einen distalen Endabschnitt aufweist, der sich an einen im Balloninnenraum angeordneten Abschnitt des Innenschafts anschließt, wobei der distale Endabschnitt des Innenschafts mit dem distalen Endabschnitt des Ballons verschweißt ist.

Erfindungsgemäß ist vorgesehen, dass der im Balloninnenraum angeordnete Abschnitt des Innenschafts mehrere übereinander liegende und miteinander verbundene Materialschichten aufweist, die ein Lumen des Innenschafts umgeben, wohingegen der distale Endabschnitt des Innenschafts einschichtig ausgebildet ist.

Im Rahmen der vorliegenden Erfindung bezeichnet der Begriff "distal" eine Komponente oder einen Bereich des Ballonkatheters, der entlang des Verlaufs des Ballonkatheters weiter entfernt ist von einer den Ballonkatheter bei der Implantation handhabenden Person (Arzt) als eine entsprechende proximale Komponente bzw. proximaler Bereich des Katheters.

Gemäß einer Ausführungsform des Ballonkatheters ist vorgesehen, dass der im Balloninnenraum (20) angeordnete Abschnitt (32) des Innenschafts (3) zumindest eine äußerste Materialschicht (3a) und eine innerste Materialschicht (3c) aufweist, wobei der distale Endabschnitt (31) des Innenschafts (3) durch die äußerste Materialschicht gebildet wird

Gemäß einer Ausführungsform des Ballonkatheters ist vorgesehen, dass der im Balloninnenraum angeordnete Abschnitt des Innenschafts eine äußerste Materialschicht aufweist, deren Verlängerung den distalen Endabschnitt des Innenschafts bildet.

Für den hierin vorgeschlagenen Ballonkatheter sind für den im Balloninnenraum angeordnete Abschnitt des Innenschafts mehrere übereinander liegende und miteinander verbundene Materialschichten vorgesehen. Dies bedeutet gemäß der vorliegenden Idee, dass zumindest zwei übereinander liegende und miteinander verbundene Materialschichten vorliegen. Die Anzahl übereinander liegenden und miteinander verbundenen Materialschichten soll grundsätzlich nur durch technische Gegebenheiten limitiert sein. Zum heutigen Tag sind beispielsweise durch Coextrusion acht übereinander liegende und miteinander verbundene Materialschichten herstellbar. In einer bevorzugten Ausführungsform weist der im Balloninnenraum (20) angeordnete Abschnitt (32) des Innenschafts (3) mindestens drei Materialschichten auf.

Weiterhin ist gemäß einer Ausführungsform des Ballonkatheters vorgesehen, dass die äußerste Materialschicht aus einem der folgenden Stoffe gebildet ist: einem Polyamid, vorzugsweise Polycaprolactam (PA 6, CAS-Nr. 25038-54-4), Poly-(*N,N'-*hexamethylenadipindiamid (Poly-(hexamethylenadipamid, PA 6.6, CAS-Nr. 32131-17-2), Poly-(hexamethylensebacamid) (PA 6.10, CAS-Nr. 9011-52-3), Poly-(hexamethylendodecandiamid) (PA 6.12, CAS-Nr. 26098-55-5), Polyundecanolactam (PA 11, CAS-Nr. 25035-04-5) und Polylauryllactam (CAS Nr. 24937-16-4), was auch als Polyamid 12 bzw. PA 12 bzw. Nylon 12 bezeichnet wird. Weitere Alternativen wären Peba Elastomere wie beispielsweise Polyetherblockamide, bevorzugt dasjenige mit CAS Nr. 77402-38-1, aber auch jene mit der Bezeichnung Pebax (Handelsname) xx SA 01 MED, wobei xx 2533, 3533, 4033, 4533, 5533, 6333, 7033, 7233 oder MV 1074 sein kann, Polyethylenterephthalat (PET, CAS Nr. 25038-59-9) und thermoplastisches Polyurethan, zum Beispiel dasjenige mit CAS Nr. 75701-44-9.Desweiteren können Polyester verwendet werden oder Polyvinylchlorid, insbesondere in Verbindung mit Weichmachern wie beispielsweise Tris(2-ethylhexyl)trimellitat mit CAS Nr. 3319-31-1.

In einer Ausführungsform kann die äußerste Materialschicht ausgewählt sein aus der Gruppe umfassend oder bestehend aus Polyamid, Polycaprolactam, Poly-(*N,N'-*hexamethylenadipindiamid, Poly-(hexamethylensebacamid), Poly-(hexamethylen-dodecandiamid), Polyundecanolactam, Polylauryllactam, Polyetherblockamid, Pebax, Polyethylenterephthalat, Polyurethan, Polyester und Polyvinylchlorid.

In einer bevorzugten Ausführungsform ist die äußerste Materialschicht aus einem hierin genannten Polyamid gebildet.

Weiterhin ist gemäß einer Ausführungsform des Ballonkatheters vorgesehen, dass der im Balloninnenraum angeordnet Abschnitt des Innenschafts eine innerste reibungsarme Materialschicht aufweist, wobei vorzugsweise die innerste Materialschicht aus einem der folgenden Stoffe gebildet ist: Polyethylen (PE), insbesondere hochdichtes Polyethylen (HDPE) wie beispielsweise mit CAS Nr. 9002-88-4, oder fluorierte Polymere, insbesondere fluorierte Polyolefine wie beispielsweise Polytetrafluorethylen (PTFE) wie beispielsweise mit CAS Nr. 9002-84-0.

Bei HDPE handelt es sich um Polyethylen mit schwach verzweigten Polymerketten, so dass eine hohe Dichte vorliegt, die insbesondere zwischen 0,94 g/cm³ und 0,97 g/cm³ liegt.

Gemäß einer weiteren Ausführungsform des Ballonkatheters ist vorgesehen, dass die innerste Materialschicht über eine mittlere Materialschicht des im Balloninnenraum angeordneten Abschnitts des Innenschafts mit der äußersten Materialschicht verbunden ist. Hierbei kann die mittlere Materialschicht die Funktion eines Haftvermittlers zwischen der äußersten und der innersten Materialschicht erfüllen.

In einer bevorzugten Ausführungsform ist die besagte mittlere Materialschicht aus einem Polyethylenhaftvermittler gebildet. Geeignete Polyethylenhaftvermittler sind vorzugsweise ausgewählt aus der Gruppe umfassend oder bestehend aus Polyethylen mit geringer Dicht (LDPE, "low density"), insbesondere und bevorzugt lineares Polyethylen mit geringer Dichte (LLDPE, linear low density polyethylen), Ethylen-basierte Copolymere wie beispielsweise Ethylen-Vinylacetat-Copolymer, wie beispielsweise das mit CAS Nr. 24937-78-8 oder Ethylen-Methylacrylat Copolymer, und polyethylenbasierte klebende Harze wie beispielsweise maleinsäureanhydrid modifizierte polyethylenbasierte klebende Harze. Beispiele für LLDPE sind Orevac 18300 (Markenname/Produktname) und Plexar PX 3080 (Markenname/Produktname). Ein Beispiel für einen polyethylenbasierten klebenden Harz ist Admer NF408E (Markenname/Produktname).

Bei den Polyethylenhaftvermittlern liegt die Dichte vorzugsweise zwischen 0,91 g/cm³ und 0,93 g/cm³.

Gemäß einer weiteren Ausführungsform des Ballonkatheters ist vorgesehen, dass der distale Endabschnitt des Ballons weiterhin mit einer Spitze des Ballonkatheters verbunden ist, die abschnittsweise aus dem distalen Endabschnitt des Ballons heraussteht, wobei die Spitze des Weiteren mit dem distalen Endabschnitt des Innenschafts verbunden ist. Gemäß einer weiteren Ausführungsform des Ballonkatheters ist vorgesehen, dass das Lumen des Innenschafts zur Aufnahme eines Führungsdrahtes ausgebildet ist, wobei der Führungsdraht insbesondere über die Spitze aus dem Ballonkatheter herausführbar ist.

Gemäß einer weiteren Ausführungsform des Ballonkatheters ist vorgesehen, dass der Ballonkatheter einen Außenschaft aufweist, der ein Lumen umgibt, wobei der Innenschaft in dem Lumen des Außenschaftes angeordnet ist und an einem distalen Endabschnitt des Außenschaftes aus dem Lumen des Außenschaftes herausgeführt ist. Insbesondere ist der Balloninnenraum über den Außenschaft mit einem fluiden Medium zum Entfalten des Ballons beaufschlagbar.

Insbesondere schließt sich der im Balloninnenraum angeordnete Abschnitt des Innenschafts an einen proximalen Abschnitt des Innenschafts an, der im Außenschaft geführt ist und sich auch außerhalb des Balloninnenraums erstrecken kann. Der proximale Abschnitt weist ebenfalls vorzugsweise die mehreren übereinanderliegenden und miteinander verbundenen Materialschichten auf.

Gemäß einer weiteren Ausführungsform des Ballonkatheters ist vorgesehen, dass der Ballon einen proximalen Endabschnitt aufweist, der mit dem distalen Endabschnitt des Außenschaftes verbunden ist.

In einer bevorzugten Ausführungsform umfasst der hierin vorgeschlagene Ballonkatheter folgende Bauteile
- einem Ballon, der einen Balloninnenraum umgibt und einen distalen Endabschnitt aufweist, und
- einem sich durch den Balloninnenraum erstreckenden Innenschaft, der einen distalen Endabschnitt aufweist, der sich an einen im Balloninnenraum angeordneten Abschnitt des Innenschafts anschließt, wobei der distale Endabschnitt des Innenschafts mit dem distalen Endabschnitt des Ballons verschweißt ist, wobei der im Balloninnenraum angeordnete Abschnitt des Innenschafts drei übereinander liegende und miteinander verbundene Materialschichten aufweist, die ein Lumen des Innenschafts umgeben, wohingegen der distale Endabschnitt des Innenschafts einschichtig ausgebildet ist.

In einer weiteren bevorzugten Ausführungsform sind die drei Materialschichten so ausgeführt, dass die äußerste Materialschicht aus Polylauryllactam (Nylon 12) und die innerste Schicht aus Polyethylen mit hoher Dichte besteht, wobei zwischen der äußersten und innersten Materialschicht ein Polyethylenhaftvermittler angeordnet ist, vorzugsweise aus LLDPE.

In einer weiteren Ausführungsform sind die drei Materialschichten so ausgeführt, dass die äußerste Materialschicht aus Polyvinylchlorid und die innerste Schicht aus Polyethylen mit hoher Dichte besteht, wobei zwischen der äußersten und innersten Materialschicht ein Polyethylenhaftvermittler angeordnet ist, vorzugsweise aus Ethylen-Vinylacetat-Copolymer.

Gemäß einem weiteren Aspekt der vorliegenden Erfindung wird ein Verfahren zur Herstellung eines Ballonkatheters, vorzugsweise eines hierin vorgeschlagenen Ballonkatheters, offenbart, aufweisend die Schritte:
- Bereitstellen eines Innenschafts des Ballonkatheters, wobei der Innenschaft mehrere übereinanderliegende und miteinander verbundene Materialschichten aufweist, die ein Lumen des Innenschafts umgeben, und
- Entfernen der Materialschichten an einem distalen Endabschnitt des Innenschafts bis auf eine äußerste Materialschicht, so dass der distale Endabschnitt des Innenschafts einschichtig ausgebildet ist.

Gemäß einer Ausführungsform des Verfahrens ist vorgesehen, dass die Materialschichten an dem distalen Endabschnitt des Innenschafts bis auf die äußerste Materialschicht mittels eines spanenden Verfahrens entfernt werden, insbesondere durch Bohren mit einem Bohrer, Reiben mit einer Reibahle oder Fräsen mit einem Fräskopf. Hierzu kann die unten beschriebene Vorrichtung verwendet werden. In einer weiteren Ausführungsform des hierin vorgeschlagenen Verfahrens ist vorgesehen, dass die Materialschichten an dem distalen Endabschnitt des Innenschafts bis auf die äußerste Materialschicht mittels einer Laserablation, vorzugsweise mittels eines Femtosekundenlasers entfernt werden.

Gemäß einer Ausführungsform des Verfahrens ist weiterhin vorgesehen, dass die äußerste Materialschicht aus einem der hierin vorgeschlagenen Stoffe gebildet ist, und vorzugsweise aus einem der folgenden Stoffe gebildet ist: einem Polyamid, insbesondere Polylauryllactam (PA 12).

Weiterhin ist gemäß einer Ausführungsform des Verfahrens vorgesehen, dass der Innenschaft eine innerste Materialschicht aufweist, wobei vorzugsweise die innerste Materialschicht aus einem der hierin vorgeschlagenen Stoffe gebildet ist, und vorzugsweise aus einem der folgenden Stoffe gebildet ist: Polyethylen (PE), vorzugsweise HDPE.

Weiterhin ist gemäß einer Ausführungsform des Verfahrens vorgesehen, dass die innerste Materialschicht über eine mittlere Materialschicht des Innenschafts mit der äußersten Materialschicht verbunden ist, wobei insbesondere die mittlere Materialschicht einen Haftvermittler zwischen der äußersten und der innersten Materialschicht bildet, und wobei insbesondere die mittlere Materialschicht aus einem der hierin vorgeschlagenen Stoffe gebildet ist, und vorzugsweise aus einem Polyethylenhaftvermittler wie beispielsweise LLDPE gebildet ist.

Vorzugsweise werden die innerste Materialschicht und die mittlere Materialschicht mittels des spanenden Verfahrens (z.B. mittels eines Bohrers, einer Reibahle oder eines Fräskopfes) so entfernt, dass der distale Endabschnitt des Innenschafts nur noch durch die äußere Materialschicht gebildet wird. Alternativ werden die innerste Materialschicht und die mittlere Materialschicht mittels Laserablation (z.B. mittels eines Femtosekundenlasers) entfernt.

Beim Entfernen der innersten und der mittleren Materialschicht kann ein Bereich der äußersten Materialschicht an einer dem Lumen des Innenschafts zugewandten Innenseite des distalen Endabschnitts des Innenschafts abgetragen so werden, dass sich der distale Endabschnitt über eine umlaufende Stufe an einen restlichen Abschnitt des Innenschafts anschließt.

Nach dem Entfernen der besagten Materialschichten am distalen Endabschnitt des Innenschafts kann der distale Endabschnitt des Ballons mit dem distalen Endabschnitt des Innenschafts verbunden werden, z.B. indem der distale Endabschnitt des Ballons auf dem distalen Endabschnitt des Innenschafts fixiert wird, z.B. mittels eines Schrumpfschlauches, und mittels eines Lasers mit dem distalen Endabschnitt des Innenschafts verschweißt wird. Alternativ dazu kann auch der distale Endabschnitt des Ballons mit dem distalen Endabschnitt des Innenschafts verbunden werden, z.B. indem der distale Endabschnitt des Ballons auf dem distalen Endabschnitt des Innenschafts fixiert wird, z.B. mittels eines Schrumpfschlauches, und mittels eines Lasers mit dem distalen Endabschnitt des Innenschafts verschweißt wird und im Anschluss daran werden die besagten Materialschichten abgetragen.

Weiterhin kann der proximale Endabschnitt des Ballons mit einem distalen Endabschnitt des Außenschaftes des Ballonkatheters verbunden werden, wobei der Innenschaft samt distalen Endabschnitt am distalen Endabschnitt des Außenschaftes aus einem Lumen des Außenschaftes herausgeführt sein kann.

Weiterhin kann der Ballon in bekannter Weise gefaltet werden. Schließlich kann in bekannter Weise ein Stent, insbesondere ein medikamentenbeschichteter Stent, auf den Ballon gecrimpt werden.

Der Stent kann später mittels des Ballonkatheters in ein Gefäß eines Patienten implantiert werden (z.B. bei einer Angioplastie), wobei der Stent durch Entfalten des Ballons aufweitbar und damit im Gefäß festlegbar ist. Zum Entfalten des Ballons kann der Balloninnenraum über den Außenschaft mit einem geeigneten fluiden Medium beaufschlagt werden.

Schließlich betrifft die Erfindung eine Vorrichtung zum Entfernen von Materialschichten eines Innenschaftes bei der Herstellung eines Ballonkatheters, insbesondere eines erfindungsgemäßen Ballonkatheters, mit:
- einem Werkzeug zum spanenden Abtragen der Materialschichten,
- einem Drehmotor zum Rotieren des Werkzeugs um eine Rotationsachse,
- einer koaxial zur Rotationsachse angeordneten Düse, wobei die Düse das Werkzeug umgibt, so dass das Werkzeug einer Düsenöffnung gegenüberliegt,
- einem mit der Düse in Strömungsverbindung stehendem Partikelsauger zum Absaugen von beim Abtragen der Materialschichten entstehenden Partikeln (bzw. Spänen),
wobei die Düse zur formschlüssigen Aufnahme des distalen Endabschnitts des Innenschafts über die Düsenöffnung ausgebildet ist, so dass beim Einführen des distalen Endabschnitts des Innenschafts in die Düse das um die Rotationsachse rotierende Werkzeug die Materialschichten des distalen Innenschafts bis auf eine äußerste Materialschicht abträgt.

Bei dem Werkzeug kann es sich z.B. um einen Bohrer, eine Reibahle oder einen Fräskopf handeln.

Bei dem Entfernen der besagten Materialschichten (insbesondere der innersten und der mittleren Materialschicht) kann ebenfalls ein Bereich der äußersten Materialschicht an einer dem Lumen des Innenschafts zugewandten Innenseite des distalen Endabschnitts des Innenschafts abgetragen werden, so dass sich der distale Endabschnitt bzw. dessen einzige (äußerste) Materialschicht über eine umlaufende Stufe an einen restlichen Abschnitts des Innenschafts anschließt.

Ein weiterer Aspekt der vorliegenden Erfindung soll ferner in der Verwendung eines Werkzeugs zum spanenden Abtragen wie beispielsweise eines Bohrers, einer Reibahle oder eines Fräskopfes oder eines Lasers, insbesondere eines Femtosekundenlasers, zum Abtragen von inneren Materialschichten eines Katheters, insbesondere mit einem maximalen Durchmesser von 0,50 mm, bevorzugt von 0.48 mm und weiter bevorzugt von 0,36 mm liegen.

Im Folgenden sollen Ausführungsformen der Erfindung sowie weitere Merkmale und Vorteile der Erfindung anhand der Figuren erläutert werden. Es zeigen:
- Fig. 1: eine schematische Schnittansicht eines erfindungsgemäßen Ballonkatheters;
- Fig. 2: eine schematische Schnittansicht eines distalen Endabschnitts eines Innenschafts des Ballonkatheters im Bereich A der Figur 1;
- Fig. 3: eine Schnittansicht der Ebene III-III gemäß Figur 2;
- Fig. 4: eine Vorrichtung zum Abtragen von Materialschichten des Innenschafts; und
- Fig. 5: eine Aufnahme eines delaminierten Bereichs eines Innenschafts.

Die vorliegende Erfindung betrifft einen Ballonkatheter 1 bzw. ein Verfahren zur Herstellung eines solchen Ballonkatheters.

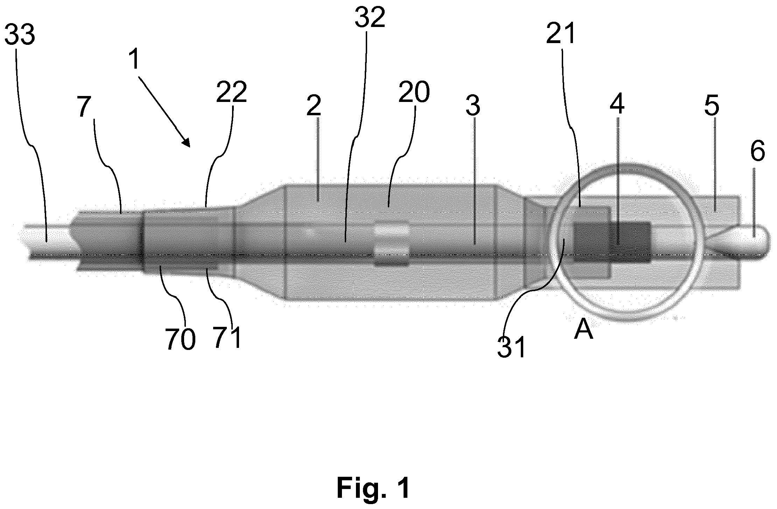

Der Ballonkatheter 1 weist gemäß der in der Figur 1 gezeigten Ausführungsform einen Ballon 2 auf, der einen Balloninnenraum 20 umgibt und einen proximalen und einen distalen Endabschnitt 22, 21 aufweist. Auf den Ballon 2 kann ein nicht gezeigter Stent gecrimpt sein. Bei dem Stent kann es sich um einen medikamentenbeschichteten Stent handeln. Der Ballonkatheter 1 dient hierbei zum Implantieren und Festlegen des Stents an der Gefäßwand eines Patienten (z.B. bei einer Angioplastie). Der Ballonkatheter 1 weist weiterhin vorzugsweise einen Außenschaft 7 auf, der ein Lumen 70 umgibt, wobei ein proximaler Abschnitt 33 des Innenschafts 3 des Katheters 1 in dem Lumen 70 des Außenschaftes 7 angeordnet ist und an einem distalen Endabschnitt 71 des Außenschaftes 7 aus dem Lumen 70 des Außenschaftes 7 herausgeführt ist, so dass sich ein weiterer Abschnitt 32 des Innenschafts 3 im Balloninnenraum 20 hin zu einem distalen Endabschnitt 31 des Innenschafts 3 erstreckt. Der Ballon 2 ist insbesondere über den proximalen Endabschnitt 22 des Ballons 2 mit dem distalen Endabschnitt 71 des Außenschaftes 7 verbunden. Weiterhin ist der Ballon 2 über seinen distalen Endabschnitt 21 mit dem distalen Endabschnitt 31 des Innenschafts 3 verbunden, insbesondere verschweißt.

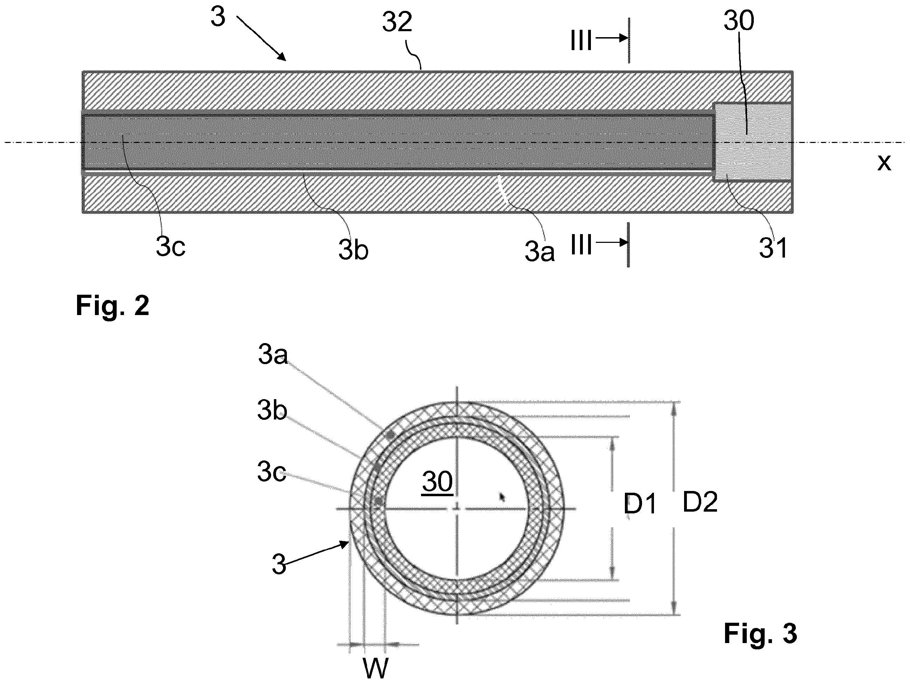

Wie beispielhaft anhand der Figuren 2 und 3 ersichtlich ist, ist vorgesehen, dass der im Balloninnenraum 20 angeordnete Abschnitt 32 des Innenschafts 3 sowie insbesondere der proximale Abschnitt 33 mehrere übereinanderliegende und miteinander verbundene Materialschichten 3a, 3b, 3c aufweisen, die ein Lumen 30 des Innenschafts 3 umgeben. Mit anderen Worten weist der Innenschaft 3 beispielhaft in den Abschnitten 32 bzw. 33 eine dreischichtige Wandung auf.

Gemäß einem Beispiel der Erfindung kann die äußerste Materialschicht 3a aus Polyamid bestehen. Die innerste Materialschicht 3c besteht dabei bevorzugt aus HDPE und die mittlere Materialschicht 3b vorzugsweise aus einem Polyethylenhaftvermittler wie LLDPE.

Der Innendurchmesser D1 des Innenschafts 3 kann z.B. 0,42 mm betragen. Der Außendurchmesser D2 kann hierbei 0,57 mm betragen. Hierbei können weiterhin die innerste und die mittlere Materialschicht 3c, 3b gemeinsam eine Stärke W von 0,023 mm aufweisen (vgl. Fig. 3).

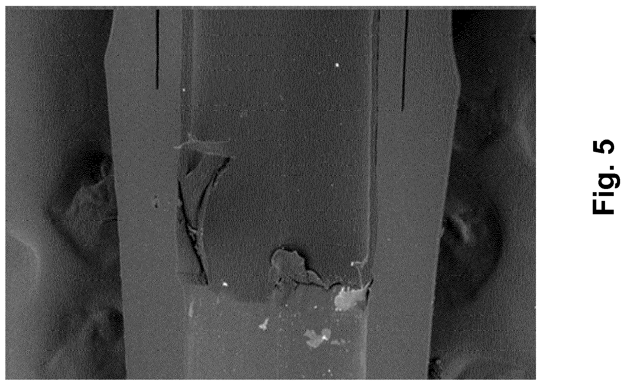

Beim Verschweißen eines derartigen Innenschafts 3 mit einem Ballon 2 kann es im Bereich der Schweißverbindung zu unerwünschten Delaminationen der Lagen 3a, 3b, 3c kommen. Dies ist in der Figur 5 anhand einer mittels eines Mikroskops erzeugten Aufnahme eines Innenschafts gezeigt.

Zum Verhindern eine derartigen Delamination sieht die Erfindung vor, den distale Endabschnitt 31 des Innenschafts 3, der mit dem distalen Endabschnitt 21 des Ballons 2 verschweißt werden soll, einschichtig herzustellen. Der distale Endabschnitt 31 des Innenschafts 3 besteht dabei vorzugsweise lediglich aus einer Schicht (3a) PA 12.

Zum Entfernen der beiden inneren Materialschichten 3b, 3c am distalen Endabschnitt 31 des Innenschafts 3 kann dieser z.B. auf einer Länge von 5 mm bis 10 mm so bearbeitet werden, dass dort nur die äußerste Materialschicht 3a stehen bleibt, beispielsweise durch bohren, spanen oder laserabtragen.

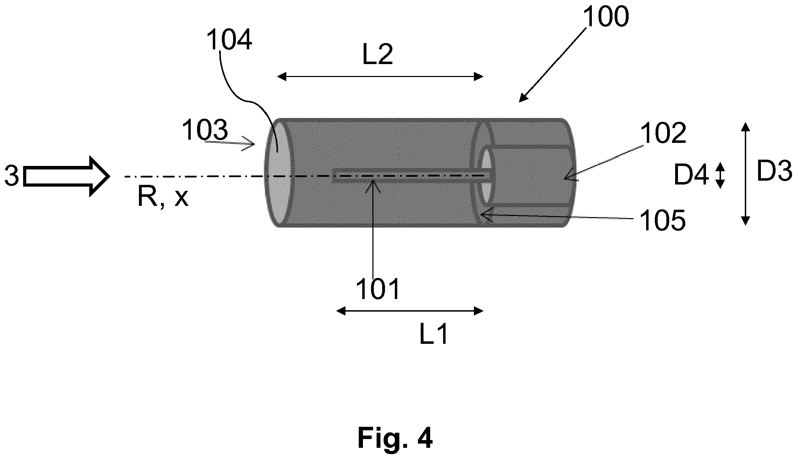

Hierzu kann eine Vorrichtung 100 gemäß Figur 4 verwendet werden. Die Vorrichtung 100 weist hierzu ein Werkzeug 101 auf, bei dem sich vorliegend z.B. um einen Bohrer oder eine Reibahle handelt, das zum spanenden Abtragen der Materialschichten 3b, 3c konfiguriert ist. Das Werkzeug 100 weist in Richtung der Rotationsachse R eine Länge L1 auf (z.B. 5 mm), die der Länge der zu entfernenden Materialschichten 3b, 3c entspricht.

Das Werkzeug 101 wird zum Abtragen der Materialschichten 3b, 3c mittels eines Drehmotors 102 in Rotation versetzt, wobei die Rotationsachse R so ausgerichtet wird, dass sie mit der Längsachse x des Katheters 1 bzw. Innenschafts 3 zusammenfällt. Weiterhin ist das Werkzeug 101 in einer koaxial zum Werkzeug 101 ausgerichteten Düse 103 angeordnet, die zum Führen des distalen Endabschnitts 31 des Innenschafts beim Abtragen der Materiallagen 3b, 3c dient.

Hierzu wird der distale Endabschnitt 31 des Innenschafts 3 über eine Düsenöffnung 104 in die Düse 103 formschlüssig eingeführt, wobei das rotierende Werkzeug 101 in den distalen Endabschnitt 31 eindringt und dort die beiden inneren Materiallagen 3b, 3c entfernt. Die Düse 103 weist zum Führen des Innenschafts 3 in Richtung der Rotationsachse R eine Länge L2 auf, die vorzugsweise größer oder gleich L1 ist.

Im Falle des oben genannten Beispiels beträgt der Durchmesser D4 des Bohrers 101 z.B. 0,47 mm bei einer Länge L1 von z.B. 5 mm und der Innendurchmesser D3 der Düse 103 beträgt z.B. 0,575 mm bei einer Länge L2 von z.B. 10 mm.

Die beim Abtragen der inneren Schichten 3b, 3c entstehenden Partikel bzw. Späne können mit einem Partikelsauer 105 der Vorrichtung 100 abgesaugt werden, der mit der Düse 103 in Strömungsverbindung steht. Diese Einrichtung stellt zusätzlich noch eine Kühlung für den Bohrer bereit.

Nach dem Entfernen der Materialschichten 3b, 3b im distalen Endabschnitt 31 des Innenschafts 3 kann dieser gefahrlos mittels eines Lasers mit dem distalen Endabschnitt 21 des Ballons 2 verschweißt werden. Hierzu kann der distale Endabschnitt 21 des Ballons 2 mittels eines Schrumpfschlauchs 5, der in der Figur 1 gezeigt ist, auf dem distalen Endabschnitt 31 des Innenschafts 3 fixiert werden.

Die Erfindung verhindert mit Vorteil Delaminationen im Bereich der Schweißverbindung zwischen den distalen Endabschnitten 21, 31 des Ballons 2 und des Innenschafts 3, wodurch das Risiko eines Blockierens eines Führungsdrahtes 6, der im Lumen 30 des Innenschafts 3 geführt wird, sinkt. Weiterhin werden verfahrensseitig eine höhere Produktivität sowie eine niedrigere Ausschussquote erzielt.

## Patentansprüche

1. Ballonkatheter (1), mit:
- einem Ballon (2), der einen Balloninnenraum (20) umgibt und einen distalen Endabschnitt (21) aufweist, und
- einem sich durch den Balloninnenraum (20) erstreckenden Innenschaft (3), der einen distalen Endabschnitt (31) aufweist, der sich an einen im Balloninnenraum (20) angeordneten (32) Abschnitt des Innenschafts (3) anschließt, wobei der distale Endabschnitt (31) des Innenschafts (3) mit dem distalen Endabschnitt (21) des Ballons (2) verschweißt ist,
**dadurch gekennzeichnet,**
**dass** der im Balloninnenraum (20) angeordnete Abschnitt (32) des Innenschafts (3) mehrere übereinander liegende und miteinander verbundene Materialschichten (3a, 3b, 3c) aufweist, die ein Lumen (30) des Innenschafts (3) umgeben, wohingegen der distale Endabschnitt (31) des Innenschafts (3) einschichtig ausgebildet ist.

2. Ballonkatheter nach Anspruch 1, wobei der im Balloninnenraum (20) angeordnete Abschnitt (32) des Innenschafts (3) zumindest eine äußerste Materialschicht (3a) und eine innerste Materialschicht (3c) aufweist, wobei der distale Endabschnitt (31) des Innenschafts (3) durch die äußerste Materialschicht gebildet wird.

3. Ballonkatheter nach Anspruch 2, wobei die äußerste Materialschicht (3a) aus einem der folgenden Stoffe gebildet ist: einem Polyamid, Polylauryllactam.

4. Ballonkatheter nach einem der vorhergehenden Ansprüche, wobei der im Balloninnenraum (20) angeordnete Abschnitt (32) des Innenschafts (3) mindestens drei Materialschichten aufweist.

5. Ballonkatheter nach einem der Ansprüche 2 bis 4, wobei die innerste Materialschicht aus einem der folgenden Stoffe gebildet ist: Polyethylen (PE), HDPE.

6. Ballonkatheter nach einem der Ansprüche 2 bis 5, wobei die innerste Materialschicht (3c) über eine mittlere Materialschicht (3b) des im Balloninnenraum (20) angeordneten Abschnitts (32) des Innenschafts (3) mit der äußersten Materialschicht (3a) verbunden ist.

7. Ballonkatheter nach Anspruch 6, wobei die mittlere Materialschicht (3b) einen Haftvermittler zwischen der äußersten und der innersten Materialschicht (3a, 3c) bildet.

8. Ballonkatheter nach Anspruch 6 oder 7, wobei die mittlere Materialschicht (3b) aus einem Polyethylenhaftvermittler gebildet ist.

9. Ballonkatheter nach einem der vorhergehenden Ansprüche, wobei der distale Endabschnitt (21) des Ballons (2) weiterhin mit einer Spitze (4) des Ballonkatheters (1) verbunden ist, die abschnittsweise aus dem distalen Endabschnitt (21) des Ballons (2) heraussteht, wobei die Spitze (4) des Weiteren mit dem distalen Endabschnitt (31) des Innenschafts (3) verbunden ist.

10. Ballonkatheter einem der vorhergehenden Ansprüche, wobei das Lumen (30) des Innenschafts (3) zur Aufnahme eines Führungsdrahtes (6) ausgebildet ist.

11. Verfahren zur Herstellung eines Ballonkatheters (1), insbesondere eines Ballonkatheters nach einem der vorhergehenden Ansprüche, aufweisend die Schritte:
- Bereitstellen eines Innenschafts (3) des Ballonkatheters (1), wobei der Innenschaft (3) mehrere übereinanderliegende und miteinander verbundene Materialschichten (3a 3b, 3c) aufweist, die ein Lumen (30) des Innenschafts (3) umgeben, und
- Entfernen der Materialschichten (3b, 3c) an einem distalen Endabschnitt (31) des Innenschafts (3) bis auf eine äußerste Materialschicht (3a), so dass der distale Endabschnitt (31) des Innenschafts (3) einschichtig ausgebildet ist.

12. Vorrichtung (100) zum Entfernen von Materialschichten (3b, 3c) eines Innenschafts (3) bei der Herstellung eines Ballonkatheters (1), insbesondere eines Ballonkatheters nach einem der Ansprüche 1 bis 13, mit:
- einem Werkzeug (101) zum spanenden Abtragen der Materialschichten (3b, 3c),
- einem Drehmotor (102) zum Rotieren des Werkzeugs (101) um eine Rotationsachse (R),
- einer koaxial zur Rotationsachse (R) angeordneten Düse (103), wobei die Düse (103) das Werkzeug (101) umgibt, so dass das Werkzeug (101) einer Düsenöffnung (104) gegenüberliegt,
- einem mit der Düse (103) in Strömungsverbindung stehendem Partikelsauger (105) zum Absaugen von beim Abtragen entstehenden Partikeln,
wobei die Düse (103) zur formschlüssigen Aufnahme des distalen Endabschnitts (31) des Innenschafts (3) ausgebildet ist, so dass beim Einführen des distalen Endabschnitts (31) des Innenschafts (3) in die Düse (103) das um die Rotationsachse (R) rotierende Werkzeug (10) die Materialschichten (3b, 3c) des distalen Endabschnitts (31) des Innenschafts (3) bis auf eine äußerste Materialschicht (3a) abträgt.
